# EUROPEAN PATENT SPECIFICATION

(11) **EP 2 309 273 B1**
(45) Date of publication and mention of the grant of the patent: **18.05.2016**
(21) Application number: 09170444.5
(22) Date of filing: 16.09.2009
(51) Int. Cl.: G01N 33/68

(54) **Novel tumor marker determination**
Neue Tumormarkiererbestimmung
Détermination de nouveau marqueur de tumeur

(43) Date of publication of application: 13.04.2011
(73) Proprietor: ZEILLINGER, Robert, 2602 Blumau-Neurisshof (AT)
(72) Inventor: Zeillinger, Robert, 2602 Blumau-Neurisshof (AT); Obermayr, Eva, 1200 Wien (AT); Cacsire Castillo-Tong, Dan, 1220 Wien (AT)
(74) Representative: Redl, Gerda

(56) References cited:
- WO-A2-2004/044154
- WO-A2-2006/018290
- US-A1- 2007 231 822
- SIEUWERTS ANIETA M ET AL: "Which cyclin E prevails as prognostic marker for breast cancer? Results from a retrospective study involving 635 lymph node-negative breast cancer patients" CLINICAL CANCER RESEARCH, THE AMERICAN ASSOCIATION FOR CANCER RESEARCH, US, vol. 12, no. 11 Part 1, 1 June 2006 (2006-06-01), pages 3319-3328, XP009125196 ISSN: 1078-0432
- PAYTON MARC ET AL: "Deregulation of cyclin E2 expression and associated kinase activity in primary breast tumors" ONCOGENE, NATURE PUBLISHING GROUP, GB, vol. 21, no. 55, 5 December 2002 (2002-12-05), pages 8529-8534, XP009125193 ISSN: 0950-9232
- GUDAS J M ET AL: "Cyclin E2, a novel G1 cyclin that binds Cdk2 and is aberrantly expressed in human cancers" 19990101, vol. 19, no. 1, 1 January 1999 (1999-01-01), pages 612-622, XP002109552
- GRESHAM DAVID ET AL: "Comparing whole genomes using DNA microarrays", NATURE REVIEWS: GENETICS, NATURE PUBLISHING GROUP, UNITED KINGDOM, vol. 9, no. 4, 1 April 2008 (2008-04-01), pages 291-302, XP002563913, ISSN: 1471-0064, DOI: 10.1038/NRG2335
- KRONICK MEL N: "Creation of the whole human genome microarray", EXPERT REVIEW OF PROTEOMICS, FUTURE DRUGS, LONDON, GB, vol. 1, no. 1, 1 June 2004 (2004-06-01), pages 19-28, XP002460172, ISSN: 1744-8387, DOI: 10.1586/14789450.1.1.19

### SEQUENCE LISTING

<110> zeillinger, Robert
<120> Novel tumor marker determination
<130> ZE3/EP
<160> 13
<170> PatentIn version 3.3
<210> 1
   <211> 22
   <212> DNA
   <213> artificial
<220>
   <223> BRCA2 sense primer
<400> 1
   ataactgaaa tcaccaaaag tg 22
<210> 2
   <211> 22
   <212> DNA
   <213> artificial
<220>
   <223> BRCA2 antisense primer
<400> 2
   ctgtagttca actaaacaga gg 22
<210> 3
   <211> 27
   <212> DNA
   <213> artificial
<220>
   <223> random primers containing a 5'-oligo-dc flanking region
<220>
   <221> misc_feature
   <222> (22)..(27)
   <223> n is a, c, g, or t
<400> 3
   cccccccccc ccccctgcag gnnnnnn 27
<210> 4
   <211> 27
   <212> DNA
   <213> artificial
<220>
   <223> CP2
<400> 4
   tcagaattca tgcccccccc ccccccc 27
<210> 5
   <211> 21
   <212> DNA
   <213> artificial
<220>
   <223> FN1 sense primer
<400> 5
   aggaaacctg ctccagtgca t 21
<210> 6
   <211> 21
   <212> DNA
   <213> artificial
<220>
   <223> FN1 antisense primer
<400> 6
   cggttggtaa acagctgcac g 21
<210> 7
   <211> 21
   <212> DNA
   <213> artificial
<220>
   <223> FN1 probe
<400> 7
   acatcgagcg gatctggccc c 21
<210> 8
   <211> 22
   <212> DNA
   <213> artificial
<220>
   <223> RBPMS sense primer
<400> 8
   caaacctcgg gagctctatc tg 22
<210> 9
   <211> 28
   <212> DNA
   <213> artificial
<220>
   <223> RBPMS antisense primer
<400> 9
   ctacaggctg tttagatgtg agctttat 28
<210> 10
   <211> 33
   <212> DNA
   <213> artificial
<220>
   <223> RBPMS probe
<400> 10
   ttttcagacc atttaagggc tatgagggtt ctc 33
<210> 11
   <211> 20
   <212> DNA
   <213> artificial
<220>
   <223> TM4SF1 sense primer
<400> 11
   ccgcttcgtg tggttctttt 20
<210> 12
   <211> 21
   <212> DNA
   <213> artificial
<220>
   <223> TM4SF1 antisense primer
<400> 12
   cagcccaatg aagacaaatg c 21
<210> 13
   <211> 23
   <212> DNA
   <213> artificial
<220>
   <223> TM4SF1 probe
<400> 13
   aggtggcctg ctgatgctcc tgc 23

## Description

The present invention relates to a method for the determination of markers of solid tumors.

A tumor marker, also called marker or biomarker, is a substance sometimes found in an increased amount in the blood, other body fluids, or tissues and which may mean that a certain type of cancer is in the body. There are many different tumor markers, each indicative of a particular disease process, and they are used in oncology as a diagnostic or prognostic marker or used to monitor cancer therapy.

Usually, tumor-specific markers are overexpressed in tumor tissue. Thus, the expression of tumour-specific genes in cancerous tissue is investigated to gain information about prognostic markers and molecular targets for diagnosis or chemical and/or immunological therapy.

*SCGB2A2*, widely known as human mammaglobin, is one of the most widely studied markers, at least in breast cancer patients. Patients are usually identified with 100% specificity. Nevertheless, mammaglobin expression is highly variable in female cancers and is detected in the blood of about 10 to 30% breast cancer patients.

Solid tumor disease is associated with carcinoma involving cancer of body tissues other than blood, bone marrow, or the lymphatic system. Surgical biopsy is called for to determine the exact nature of a solid tumor, which is a tedious and painful procedure.

Almost two million women in the whole world are diagnosed with gyneocological cancer, such as breast, cervical, endometrial or ovarian cancer each year. These gynecologic diseases contribute to 45% of female malignancies and cause about 880000 deaths in women annually. Although several advances have been made in early diagnosis during the past few decades, many patients still die of metastasis being the main cause for tumor-related death. In these patients hematogenous spreading of malignant cells remained undetected at the time of initial therapy.

Tumor cells circulating in the blood of cancer patients, also called circulating tumor cells (CTC) or disseminated tumor cells (DTC), have been described for a series of solid tumor disease, such as colorectal, lung, kidney, squamous oesophageal, liver, prostate and pancreatic malignancies. Among gynecological malignancies, most of the research has been done on CTC in breast cancer, whereas only a few data exist on CTC in ovarian, cervical and endometrial cancer. Christofanilli et al. (J Clin Oncol, 2005. 23(7): p. 1420-30) showed that the detection of CTC can predict patient outcome, and the presence of tumor cells in the peripheral blood was considered to be established as an additonal staging parameter. For these reasons many efforts have been made to develop reliable procedures for the sensitive and specific detection of CTC, either at the protein level, e.g. antibody-based cell staining, or at the mRNA level, e.g. reverse transcription PCR. While the first approach is the gold standard technique for the detection of tumor cells in the bone marrow of breast cancer patients, the latter is supposed to be more sensitive and amenable to high-throughput analysis.

Klein CA (Adv. Cancer Res. 2003; 89:35-67) describe that DTC are seldom derived from dominant clones of primary tumors. In contrast, it appears that cancer cell evolution explores a multitude of variant cells from which systemic cancer can develop independently. Thus, markers derived from studying the expression profile of tumor tissue would usually not be determined in blood samples.

The eukaryotic cell cycle is regulated by a family of serine/threonine protein kinases known as cyclin-dependent kinases (CDKs). Cyclin-dependent kinase (CDK)2 interacting cyclins perform essential functions for DNA replication and cellular proliferation. The human genome encodes two E-type cyclins (E and E2; E2 is also called CCNE2) and two A-type cyclins (A1 and A2). Dysregulation of the CDK2-bound cyclins plays an important role in the pathogenesis of cancer. Cyclin A2 is associated with cellular proliferation and can be used for molecular diagnostics as a proliferation marker. In addition, cyclin A2 expression is associated with a poor prognosis in several types of cancer.

Sieuwerts et al (Clin Cancer Res. 2006 Jun 1;12(11 Pt 1):3319-28) measured mRNA transcripts of full-length and splice variants of cyclin E1 (CCNE1) and cyclin E2 (CCNE2) by real-time PCR in frozen tumor samples from 635 lymph node-negative breast cancer patients. Both CCNE1 and CCNE2 were found to qualify as independent prognostic markers for lymph node-negative breast cancer patients; CCNE1 would provide additional information for specific subgroups of patients.

Payton et al. (Oncogene 2002; 21(55): 8529-8534) disclose the determination of Cyclin E2 expression in primary tumors.

Gudas et al. (Molecular and Cellular Biology 1999; 612-622) describes aberrant expression of Cyclin E2 in tumor cells.

US2007/0231822A1 discloses methods for the detection and treatment of cancer and a series of markers that are overexpressed in lymph node tissue. According to an example of advanced prostate cancer, the only marker that was found to be elevated in a sample of peripheral blood was the EpCAM marker. All other markers tested were not elevated.

The object of the present invention was to find new biomarkers to determine CTC in patients, which would be qualifying a solid tumor disease.

The object is achieved by the provision of the embodiments of the present invention.

### Summary of the Invention

The present invention refers to a method of determining CCNE2 in a body fluid sample of patients at risk of solid tumor disease. Specifically, the invention provides for a method of detecting CCNE2 in a body fluid sample of a patient for detecting susceptibility to breast, ovarian, endometrial, or cervical cancer, comprising
a. testing the CCNE2 nucleic acid expression in a tumor cell-enriched blood sample from said patient, and
b. comparing the result with a reference level, wherein an elevated level is an indication that the patient is at risk of a tumor disease which is breast cancer, ovarian cancer, endometrial cancer, or cervical cancer.

The preferred method according to the invention provides for the comparison of the results of determination, such as a detection parameter, with a reference value or level. A preferred embodiment comprises a comparative gene expression analysis.

In a preferred method according to the invention, at least one further marker selected from the group of DKFZp762E1312, EMP2, MAL2, PPIC, SLC6A8, GTF2IRD1, AGR2, FXYD3, S100A16, TFF1, mammaglobin A, FN, Epcam, tm4sf and rbpms is determined.

Samples from patients at risk of a solid tumor disease are preferably taken from patients who are actually suffering from cancer, in particular who have been diagnosed with cancer. Preferably samples from early stage cancer patients are determined.

In a preferred method according to the invention, the marker expression is determined. Preferably the nucleic acid and/or protein expression of the marker is determined.

In a preferred method according to the invention, the detection limit is less than 30 tumor cells/ml body fluid, such as whole blood, preferably less than 15 tumor cells/ml, preferably at least 2 tumor cells/ml whole blood.

The method according to the invention is particularly useful for the preparation of an expression pattern used for tumor stage determination.

Means for determining the expression pattern or expression signature according to the invention employ an inventive multi-marker panel, which may be used for detecting circulating tumor cells in a subject at risk of malignancy, consisting of the biomarkers CCNE2, DKFZp762E1312, EMP2, MAL2, PPIC, SLC6A8 and GTF2IRD1, and optionally one or more markers selected from the group consisting of AGR2, FXYD3, S100A16, TFF1, mammaglobin A, FN, Epcam, tm4sf and rbpms.

According to the invention there is further provided a set of reagents for detecting circulating tumor cells in a subject at risk of malignancy, consisting of reagents specifically binding to CCNE2, DKFZp762E1312, EMP2, MAL2, PPIC, SLC6A8 and GTF2IRD1, and optionally further one or more markers selected from the group consisting of AGR2, FXYD3, S100A16, TFF1, mammaglobin A, FN, Epcam, tm4sf and rbpms.

The set of reagents according to the invention preferably comprises ligands, such as antibodies or antibody fragments, which are optionally labelled.

### Detailed Description

### Cyclin E2 (CCNE2)

The eukaryotic cell cycle is regulated by serine/threonine protein kinases known as cyclin dependant kinases (CDKs). CDKs are activated by association with a cyclin regulatory subunit. Family members of the cyclin dependant kinases are CDK1, 2, 4, 5 and 6; they associate with cyclines A, B1, B2, D1-D3 and E. The formation of cyclin-CDK complexes, which needs to be phosphorylated at a conserved threonine residue for complete activity, controls the progression through the first gap phase (G₁) and initiation of DNA synthesis (S phase). The activity of cyclin-CDK complexes is negatively regulated by further phosphorylation of a tyrosin and threonine and by association with cyclin dependant kinase inhibitors.

In the last decade a second cyclin E family member, cyclin E2 was discovered. The cyclin E2 mRNA contains an open reading frame encoding a 404 amino acid protein with a calculated molecular weight of 47 kDa. The encoded protein shares 47% overall similarity to human cyclin E1 and contains a cyclin box motif that is characteristic of all cyclins but is slightly divergent from the conserved MRAILL sequence. Cyclin E2 associates with CDK2 in a functional, catalytically active kinase complex, which phosphorylates histone H1 and the retinoblastome protein Rb, but not p53. Quiescent cells contain negligible levels of the active complex, but as they approach S-phase, the kinase activity peaks followed by a gradual decline through S-phase. The ability of the cyclin E2-CDK2 complex to phosphorylate target substrates is inhibited by the CDK inhibitors p27^{Kip1} and p21^{Cip1}.

It has been shown that over-expression of cyclins decreases the length of G1 and increases the proportion of cells in S-phase. Similarly, ectopic expression of cyclin E2 also accelerates the cell cycle. Cyclin E2 mRNA levels are undetectable in normal quiescent cells arrested in G₀ and increases dramatically upon re-stimulation peaking at 12 hours with a gradual decrease into late S-phase. In contrast it has be shown that transformed cells show a prolonged cyclin E2 expression in S-phase. Also, there is evidence that the increased expression of cyclins correlates with the development of many types of human tumors. It is also known that expression of either of the papilloma virus E6 and E7 oncoproteins, which inactivate p53 and Rb, respectively, upregulates the expression of cyclin E2. It has been demonstrated that the cyclin E2 transcript is often present at elevated levels in human primary tumors compared to normal adjacent tissue and that cyclin E2 may contribute to the pathogenesis of breast cancer. Furthermore, CCNE2 may serve as independent prognostic markers for lymph node-negative breast cancer patients. A study investigating the expression of *cyclin E2* in the bone marrow from patients with acute leukemia revealed that *cyclin E2* may be used as a marker for examination of minimal residual disease in acute leukemia (Wang, Y., et al., Expressions of cyclin E2 and survivin in acute leukemia and their correlation. Zhongguo Shi Yan Xue Ye Xue Za Zhi, 2006. 14(2): p. 337-42).

### Epithelial membrane protein 2 (EMP2)

The *EMP2* cDNA generates a 18-kD protein in vitro. The EMP2 protein shares 43% amino acid identity with peripheral myelin protein-22 (PMP22); they are particularly homologous in their transmembrane domains. Due to the high amino acid sequence homology among PMP22, EMP1, EMP2, and EMP3, these proteins were assigned to a novel family. Based on the suggested functions of PMP22, EMP2 was proposed to be involved in cell proliferation and cell-cell interactions. EMP2 plays a critical role in selective receptor trafficking, affecting molecules that are important in growth control, invasion and metastasis.

Prominent *EMP2* expression was found in adult ovary, heart, lung, and intestine and lower expression in most other tissues, including the liver, whereas in the fetus, high *EMP2* mRNA levels were measured in the lung and kidney and lower levels in the liver and brain. *EMP2* is up-regulated in secretory endometrium at the window of implantation and is required for blastocyst implantation. Due to the physiologic regulation of *EMP2* in the endometrium and its role in cell-cell interaction and extracellular matrix adhesion, it was suggested that *EMP2* may play a role in endometrial carcinogenesis.

### Mal, T-cell differentiation protein 2 (MAL2)

*MAL2* was detected in the last decade as a novel member of the MAL proteolipid family. The gene encodes a 19-kD multispan transmembrane protein, which is a component of lipid rafts and which, in polarized cells, primarily localizes to endosomal structures beneath the apical membrane. The protein is required for transcytosis, an intracellular transport pathway used to deliver membrane-bound proteins and exogenous cargos from the basolateral to the apical surface. *MAL2* is a heterologous partner for proteins encoded by all three tumor protein D52-like genes and is most closely related to MAL, the first member of the MAL proteolipid family to be identified.

Interestingly the *MAL2* gene is located on chromosome 8q.23, a region frequently increased in copy number in breast and other type of cancers. One of the most important target genes affected by gains and amplifications of 8q is the *MYC* oncogene, and *CCNE2* is also located in this region.

### Solute carrier family 6 (neurotransmitter transporter, creatine), member 8 (SLC6A8)

The *SLC6A8* gene encodes for the creatine transporter which is a member of the solute carrier 6 (SLC6) family or Na⁺- and Cl⁻-dependant neurotransmitter transporters. The membrane-bound SLC6A8 protein transports creatine into the cell, which is converted into phosphocreatine by creatine kinase. Phosphocreatine can be used as a quick source of ATP production in tissues with high energy demand.

*SLC6A8* was assigned to chromosome Xq28, contains 13 exons and spans about 8.5 kb of genomic DNA. Mutations cause an X-linked creatine deficiency syndrome resulting in mental retardation, speech and language delay, autistic-like behavior and epilepsy.

Until now, the role of *SLC6A8* in cancerous diseases remains to be elucidated. The gene has been associated with the platinum pathway and it was identified that the single nucleotide polymorphism *rs11236836* among several other genetic variants was contributing to the cisplatin-induced cytotoxicity. Cisplatin is a platinum containing chemotherapeutic drug for the treatment of a variety of cancers. Also, the loss of the inactive X chromosome and loss of heterozygosity, frequent phenomena in tumorigenesis, might cause over-expression of *SLC6A8.*

### Hypothetical protein DKFZp762E1312 (DKFZp762E1312)

The hypothetical protein DKFZp762E1312 also known as Holliday junction recognition protein (HJURP), 'fetal liver expressing gene 1', and as 'up-regulated in lung cancer 9'. The activation of this novel gene seemed to play an important role in the immortality and chromosomal stability of cancer cells.

The *DKFZp762E1312* gene located on chromosome 2q37.1 encodes for an 83 kDa protein which is up-regulated in various cancer cell lines of lung and other organs. Overexpression of DKFZp762E1312 protein is observed in many lung cancer samples, compared with normal lung and is associated with poor prognosis as well. It has been shown, that DKFZp762E1312 is involved in the homologous recombination pathway in the repair processes of DNA double-strand breaks through interaction with hMSH5 and NBS1.

### Peptidyl-prolyl-isomerase C (PPIC)

The protein encoded by this gene located on chromosome 5 is a member of the peptidyl-prolyl cis-trans isomerase (PPlase) family. PPlases catalyze the cis-trans isomerization of proline imidic peptide bonds in oligopeptides and accelerate the folding of proteins. Similar to other PPlases, this protein can bind immunosuppressant cyclosporin A. Hence, they play a crucial role in the regulation of T-cell function and inflammation.

### FXYD3

This gene belongs to a small family of FXYD-domain containing regulators of Na+/K+ ATPases which share a 35-amino acid signature sequence domain, beginning with the sequence PFXYD, and containing 7 invariant and 6 highly conserved amino acids. This gene encodes a cell membrane protein that may regulate the function of ion-pumps and ion-channels. This gene may also play a role in tumor progression. Alternative splicing results in multiple transcript variants encoding distinct isoforms.

It was found that *FXYD3* in pancreatic cancer may contribute to the proliferative activity of this malignancy and that expression of *FXYD3* is an independent prognostic factor in rectal cancer patients. It is widely accepted that *FXYD3* plays an important role in cellular growth of prostate carcinomas and that this gene contains the potential to serve as a prostate cancer expression marker and. It has been shown that FXYD is highly expressed in breast cancers and responsible for cancer cell proliferation.

### TFF1

Members of the trefoil family are characterized by having at least one copy of the trefoil motif, a 40-amino acid domain that contains three conserved disulfides. They are stable secretory proteins expressed in gastrointestinal mucosa. Their functions are not defined, but they may protect the mucosa from insults, stabilize the mucus layer, and affect healing of the epithelium. This gene, which is expressed in the gastric mucosa, has also been studied because of its expression in human tumors. This gene and two other related trefoil family member genes are found in a cluster on chromosome 21. *TFF1* expression is correlated with steroid receptor status and elevated transcript levels have been observed in various neoplastic tissues, including breast cancer.

### AGR2

Human *AGR2* is a homolog of the secreted Xenopus laevis protein (XAG-2). In Xenopus, XAG-2 is primarily involved in the induction and differentiation of the cement gland, as well as in the patterning of anterior neural tissues. AGR2 has been identified as a potential marker for detection of circulating tumor cells in the blood of patients with metastatic cancers.

### S100A16

Calcium binding proteins of the S100 family play central roles in many intra- and extra-cellular processes. *S100A16* is prevalently expressed in breast cancer derived CTCs and up-regulation has been observed in many tumors, suggesting a central cellular function related to malignant transformation.

### SCGB2A2 (Mammaglobin A)

*SCGB2A2*, widely known as mammaglobin or mammaglobin A, is a member of the secretoglobin subfamily, a group of small, secretory, rarely glycosylated, dimeric proteins mainly expressed in mucosal tissues, and that could be involved in signalling, the immune response, chemotaxis and possibly, as a carrier for steroid hormones in humans.

*SCGB2A2* expression has rarely been found in healthy individuals. Thus, it has become the most widely studied marker in DTC detection after *CK19,* at least in breast cancer patients. At the same sensitivity as *CK19,* patients are identified with 100% specificity. Nevertheless, mammaglobin expression is highly variable in female cancers and is detected in the blood of only 10 to 30% breast cancer patients. Unfortunately, the most aggressive, steroid receptor-negative, high grade breast tumors and their corresponding CTC are likely to escape detection using *SCGB2A2* as marker.

*SCGB2A2* was found to be abundantly expressed in tumors of the female genital tract, i.e. endometrial, ovarian and cervical cancer. This observation might extend the diagnostic potential of *SCGB2A2* to the detection of CTC from gynecological malignancies.

### RBPMS (RNA binding protein with multiple splicing)

This gene encodes a member of the RRM family of RNA-binding proteins. The RRM domain is between 80-100 amino acids in length and family members contain one to four copies of the domain. The RRM domain consists of two short stretches of conserved sequence called RNP1 and RNP2, as well as a few highly conserved hydrophobic residues. The protein encoded by this gene has a single, putative RRM domain in its N-terminus. Alternative splicing results in multiple transcript variants encoding different isoforms.

*RBPMS* was found to be among the 20 most significantly upregulated genes both in hepatocarcinoma with high grading and with loss of 13q, which are all involved in cell-cycle control and proliferation. These findings are of clinical interest, because morphological grading has been shown to correlate with survival of patients with hepatocarcinoma, and dedifferentiation occurs in more than half of these patients within 7-34 months. Zeillinger et al. analyzed expression levels of *RBPMS* and 18 further genes in the blood of 64 ovarian cancer patients with quantitative RT-PCR following tumor cell enrichment and pre-amplification of cDNA. Detectable *RBPMS* mRNA levels were found in 30% of the patients, who had detectable mRNA levels of any of the analyzed genes (WO2006018290).

### TM4SF1 (transmembrane 4 L six family member 1)

The protein encoded by this gene is a member of the transmembrane 4 superfamily, also known as the tetraspanin family. Most of these members are cell-surface proteins that are characterized by the presence of four hydrophobic domains. The proteins mediate signal transduction events that play a role in the regulation of cell development, activation, growth and motility. This encoded protein is a cell surface antigen and is highly expressed in different carcinomas. Members of the transmembrane-4 superfamily (TM4SF) of surface proteins have been implicated in the regulation of cancer cell metastasis, and the expression of several *TM4SF* members on tumor cells is inversely correlated with patient prognosis. *TM4SF1* is expressed in most epithelial cell carcinomas and is a target for antibody mediated therapy. *TM4SF1* was suggested as a tool for diagnosing circulating tumor cells in these patients.

Although weak expression was detected in normal vascular endothelium, strong expression was found in the vascular endothelium of human cancers. Thus, *TM4SF1* might be an attractive target for antiangiogenesis therapy.

WO2006018290A2 discloses expression levels of *TM4SF1* and 18 further genes in the blood of 64 ovarian cancer patients with quantitative RT-PCR following tumor cell enrichment and pre-amplification of cDNA. Detectable *TM4SF1* mRNA levels were found in 61% of the patients, who had detectable mRNA levels of any of the analyzed genes.

### EPCAM (Epitheial cell adhesion molecule)

This gene encodes a carcinoma-associated antigen and is a member of a family that includes at least two type I membrane proteins. This antigen is expressed on most normal epithelial cells and gastrointestinal carcinomas and functions as a homotypic calcium-independent cell adhesion molecule. Because of its ubiquitous expression on the surface of epithelial cells, *EPCAM* can be considered as a pancarcinoma tumor marker. The antigen is being used as a target for immunotherapy treatment of human carcinomas.

*EPCAM* has been frequently used as target for positive immunomagnetic separation to enrich tumor cells for RT-PCR analysis. Monoclonal antibodies against this antigen have been extensively developed for diagnostic (CellSearch), but also therapeutic, approaches. Although highly sensitive for epithelial malignancies, including breast cancer, its use for CTC detection is, however, hampered by the fact that it is expressed in low amounts in peripheral blood cells. Furthermore, it has been shown that the normal-like breast cancer cells characterized by aggressive behaviour and worse treatment options are not recognized by the Veridex CellSearch test, which is the only diagnostic test for circulating tumor cells currently approved by the US Food and Drug Administration and which utilizes an anti-EpCAM antibody.

### FN1 (FN or Fibronectin 1)

This gene encodes fibronectin, a glycoprotein present in a soluble dimeric form in plasma, and in a dimeric or multimeric form at the cell surface and in extracellular matrix. Fibronectin is involved in cell adhesion and migration processes including embryogenesis, wound healing, blood coagulation, host defense, and metastasis. The gene has three regions subject to alternative splicing, with the potential to produce 20 different transcript variants. However, the full-length nature of some variants has not been determined.

Tumor growth and invasion are not only the result of malignant transformation but also depend on environmental influences from their surrounding stroma, local growth factors, and systemic hormones. In particular, the composition of the extracellular matrix is believed to affect malignant behavior in vivo. Fibronectin, a matrix glycoprotein expressed in several carcinoma cell types, has been implicated in carcinoma development.

WO2006018290A2 discloses expression levels of *FN1* and 18 further genes in the blood of 64 ovarian cancer patients with quantitative RT-PCR following tumor cell enrichment and pre-amplification of cDNA. Detectable RBPMS mRNA levels were found in 61% of the patients, who had detectable mRNA levels of any of the analyzed genes.

### GTF2I repeat domain containing 1 (GTF2IRD1)

The protein encoded by this gene contains five GTF2I-like repeats and each repeat possesses a potential helix-loop-helix (HLH) motif. It may have the ability to interact with other HLH-proteins and function as a transcription factor or as a positive transcriptional regulator under the control of Retinoblastoma protein. This gene is deleted in Williams-Beuren syndrome, a multisystem developmental disorder caused by deletion of multiple genes at 7q11.23. Alternative splicing of this gene generates at least 2 transcript variants.

CCNE2 was surprisingly found as a new candidate gene, which is overexpressed in CTC. Expression was, for instance, determined by the quantitative reverse transcriptipion PCR (qRT-PCR)-based, for the detection of CTC in the peripheral blood of patients suffering from solid tumors, such as gynecological malignancies. The CCNE2 gene is hardly expressed in the peripheral blood of healthy females but appeared very highly expressed in tumor cells. A set of differentially expressed genes was found in the cell lines, in primary tumor tissues, and surprisingly also in tumor cell-enriched blood samples taken from cancer patients as well as from healthy women processed equally as the patients' blood samples. Based on the results of these experiments, a panel of promising candidate genes was selected for routine diagnosis of disseminated tumor cells.

Many tissues containing actively dividing cells have detectable levels of cyclin E2, which is generally accepted as proliferation marker. Cyclin E2 mRNA levels are usually undetectable in arrested cells or dormant cells. It was thus surprising that CTC of solid tumors would overexpress CCNE2, indicating minimal residual disease. It was the more suprising, because CCNE1 was not found to be a differentiating biomarker. Therefore, the preferred method according to the invention would not provide for the determination of CCNE1 in blood samples of the tumor patients.

A patient at risk of solid tumor disease is herein understood as a subject that potentially develops a solid tumor disease or already suffers from such a disease at various stages, including the early stage and advanced disease state.

The term "patients" herein always includes healthy subjects. The subject can, e.g., be any mammal, in particular a human, but also selected from animals, such as those used for tumor models and other animal studies.

Preferably those patients are tested for the biomarker according to the invention, before a solid tumor is detected, or before malignancy has proven by biopsy, where no cancerous disease is diagnosed.

Healthy patients are currently not tested for any tumor disease biomarkers in the absence of any detectable tumor. However, there are patients, who have the potential to develop a solid tumor disease because of a genetic predisposition. Antecedent diseases, such as cancer, or benign tumors or certain medical treatment would also increase the risk of developing solid tumors and associated disease conditions. Several risk factors for solid tumors have been identified so far, among them BRCA1-, BRCA2-, p53- gene mutations, hormonal therapies, etc.

Thus, the present invention provides the CCNE2 marker alone, or with one or more members of a panel of biomarkers that can be used in a method for detection or diagnosis of the solid tumor disease. The markers can be used for diagnosis, in particular early stage diagnosis of respective solid tumor disease.

The early detection of solid tumor disease is essential in the patient population that is already classified as high-risk patients. It is thus preferred to test a patient population according to the invention, which is already classified as risk patients.

In particular, the inventive method allows the early stage determination of the solid tumor disease or respective risk stages, e.g. to distinguish between low, medium and high risk patients.

The multimarker panel contains or consists of CCNE2, MAL2, DKFZp762E1312, EMP2, PPIC, SLC6A8 and GTF2IRD1, and optionally further AGR2, FXYD3, S100A16, TFF1, mammaglobinA, FN, Epcam, tm4sf and rbpms. In a preferred embodiment the CCNE1 biomarker is not included in the panel.

Preferred marker combinations can be derived from the examples below, which are reaching a ratio of positive patients of at least 15%, preferably at least 20%, 30%, 40%, 50%, 60%, 70% or even more preferred at least 80%, which is for example reached by the multimarker panel of CCNE2, DKFZp762E1312, EMP2, MAL2, PPIC, SLC6A8. Likewise, any combination of at least CCNE2 and optionally one or more markers of the multimarker panel according to the invention with another marker associated with cancer, which brings about a ratio of positive patients as described above, is considered a preferred combination to determine the risk of cancer.

In a specific embodiment, the invention contemplates marker panels containing or consisting essentially of at least two, three, four, five or six or more, preferably including all of the sixteen biomarkers of the inventive panel, or consisting of these sets, wherein at least one of the biomarkers is CCNE2. The inventive panel preferably includes only those biomarkers that are associated with solid tumor disease, preferably only those that would differentiate between patients having detectable CTCs associated with malignancy and healthy subjects, which eventually have epithelial cells in a body fluid sample. The multimarker panel preferably comprises the biomarker polypeptide or gene sets.

The set of reagents according to the invention is preferably provided to determine the biomarker panel according to the invention.

CCNE2 and eventual further biomarkers are preferably determined by testing for the respective polypeptides and/or polynucleotides. In the following, biomarker or marker determination according to the invention always refers to the detection and/or testing for CCNE2 and optionally one or more markers of the multimarker panel of the invention.

The term "detect" or "detecting" includes assaying, imaging or otherwise establishing the presence or absence of the target biomarker, variants such as splice variants, subunits thereof, or combinations of reagent bound targets.

The marker expression is determined either as polynucleotide, e.g. as mRNA, or expressed polypeptide or protein. The comparison with the reference value should be of the same sample type. Thus, the reagents preferably comprise ligands specifically binding to the biomarker polypeptide or gene or genetic marker, e.g. comprising a plurality of respective polypeptides, genes or polynucleotides. Ligands are herein understood as marker specific moieties.

Marker specific moieties are substances which can bind to or detect at least one of the markers for a detection method described above and are in particular marker nucleotide sequence detecting tools or marker protein specific antibodies, including antibody fragments, such as Fab, F(ab), F(ab)', Fv, scFv, or single chain antibodies. The marker specific moieties can also be selected from marker nucleotide sequence specific oligonucleotides, which specifically bind to a portion of the marker sequences, e.g. mRNA or cDNA, or are complementary to such a portion in the sense or complementary anti-sense, like cDNA complementary strand, orientation.

The preferred ligands may be attached to solid surfaces to catch and separate the marker or CTC in the sample, and/or to labels. Biological assays require methods for detection, and one of the most common methods for quantitation of results is to conjugate a detectable label to a protein or nucleic acid that has affinity for one of the components in the biological system being studied. Detectable labels may include molecules that are themselves detectable (e.g., fluorescent moieties, electrochemical labels, metal chelates, etc.) as well as molecules that may be indirectly detected by production of a detectable reaction product (e.g., enzymes such as horseradish peroxidase, alkaline phosphatase, etc.) or by a specific binding molecule which itself may be detectable (e.g., biotin, digoxigenin, maltose, oligohistidine, 2,4-dintrobenzene, phenylarsenate, ssDNA, dsDNA, etc.).

In particular aspects of the invention, the methods described herein utilize CCNE2 and optionally one or more markers of the multimarker panel of the invention placed on a microarray so that the expression status of each of the markers is assessed simultaneously. In an embodiment, the invention provides a microarray comprising a defined set of marker genes, whose expression is significantly altered by a risk of cancer. The invention further relates to the use of the microarray as a prognostic tool to predict disease conditions associated with solid tumors.

In preferred embodiments, the mRNA concentration of the marker(s) is determined. To this extent, mRNA of the sample can be isolated, if necessary, after adequate sample preparation steps, e.g. tumor cell enrichment and/or lysis, and hybridized with marker specific probes, in particular on a microarray platform with or without amplification, or primers for PCR-based detection methods, e.g. PCR extension labelling with probes specific for a portion of the marker mRNA. In preferred embodiments the marker(s) or a combination thereof is (are) determined using a microarray with specific probes for determining CCNE2 and preferably one or more of the multimarker panel according to the invention.

Differential expression, e.g. compared to the control of healthy patients or patients suffering from a benign tumor, is preferably determined by microarray, hybridization or by amplification of the extracted polynucleotides. The invention preferably contemplates a gene expression profile comprising a multimarker panel that is associated with gyneocological cancer. This profile provides a highly sensitive and specific test with both high positive and negative predictive values permitting diagnosis and prediction of the patient's risk of developing cancer.

For example, the invention provides the method comprising
(a) contacting the body fluid sample obtained from said patient with one or more oligonucleotides that hybridize with one or more markers, which are CCNE2 and optionally one or more of the markers of the multimarker panel according to the invention, and
(b) detecting in the sample a level of polynucleotides that hybridize to the one or more markers relative to a reference level or predetermined cut-off value, and therefrom determining the risk of solid tumor disease in the patient.

Within certain preferred embodiments, the amount of mRNA is detected via polymerase chain reaction using, for example, oligonucleotide primers that hybridize to a marker gene, or complements of such polynucleotides. When using mRNA detection, the method may be carried out by combining isolated mRNA with reagents to convert to cDNA according to standard methods and analyzing the products to detect the marker presence in the sample. Within other embodiments, the genomic nucleic acid may be analyzed for the specific marker expression.

In further embodiments the amount of a marker or any combination thereof is determined by the polypeptide or protein concentration of the marker(s), e.g. with marker specific ligands, such as antibodies or specific binding partners. The binding event can, e.g., be detected by competitive or non-competitive methods, including the use of labelled ligand or marker specific moieties, e.g. antibodies, or labelled competitive moieties, including a labelled marker standard, which compete with marker proteins for the binding event. If the marker specific ligand is capable of forming a complex with the marker, the complex formation indicates expression of the markers in the sample.

In particular, the invention relates to the method comprising
(a) reacting the sample with one or more binding agents specific for CCNE2 and optionally one or more markers of the multimarker panel according to the invention, e.g. an antibody or antibody fragment that is directly or indirectly labelled with a detectable substance, and
(b) detecting the detectable substance.

The preferred method employs an immunoassay. In general, immunoassays involve contacting a sample potentially containing a biomarker of interest with at least one immunoligand that specifically binds to the marker. A signal is then generated indicative of the presence or amount of complexes formed by the binding of polypeptides in the sample to the immunoligand. The signal is then related to the presence or amount of the marker in the sample. Immunoassays and respective tools for determining CCNE2 and the other markers are well-known in the art.

The invention also relates to kits for carrying out the methods of the invention.

The invention further contemplates the methods, compositions, and kits described herein using additional markers associated with epithelial cancer. The methods described herein may be modified by including reagents to detect the additional markers, or polynucleotides for the markers.

Reference values for the biomarker are preferably obtained from a control group of patients or subjects with normal expression of said biomarker, or a biomarker expression, that is associated with the disease condition, such as disease stages, which represents the appropriate reference value. In a particular aspect, the control comprises material derived from a pool of samples from normal patients. The normal levels of a biomarker are determined in samples of the same type obtained from control patients. Elevated levels of the biomarker relative to the corresponding normal levels is an indication that the patient is at risk of solid tumor disease. The level of biomarkers or amount of biomarkers is herein understood to always refer to either the respective polypeptides or nucleotide sequence.

The risk of solid tumor disease is indicated if the amount of the biomarker or the combination of markers exceeds at least two, preferably three, standard deviations of the reference value of subjects not suffering from solid tumor disease, preferably being subjects from a control group or healthy subjects. If at least two biomarkers of the panel according to the invention are increased, the risk is considered to be increased as well.

If more than one marker is detected, the comparison is made to each single reference value for each marker in the reference itself. The inventive prognosis method can predict whether a patient is at risk of developing solid tumor disease, such as cancer. The higher the fold increase, the higher is the patient's risk of cancer. An elevated CCNE2 value alone or in combination with the other markers of the panel according to the invention indicates, for example, special treatment of the patient, using appropriate medication or further diagnostic techniques, such as imaging and surgical interventions. The method of the invention can thus be used to evaluate a patient before, during, and after medical treatment.

Likewise, the marker level can be compared to a cut-off concentration and the solid tumor disease development potential is determined from the comparison; wherein marker concentrations above the reference concentrations are predictive of cancer development in the patient.

Thus, the preferred method according to the invention comprises the step of comparing the marker level with a predetermined standard or cut-off value, which is preferably at least 25% higher than the standard, more preferred at least 40% or 50% higher, but can also be at least 100% higher.

According to a specific embodiment the numbers of CTC in the body fluid is determined. When a ligand specifically binding to the biomarker is used as capturing agent, the CTC may be enriched, optionally isolated and determined, e.g. according to their epithelial cell functions or properties.

The CTC may be enriched in the body fluid and the expression profile of the cells is determined. For example, disseminated, circulating tumour cells from peripheral blood are enriched using a cell separation procedure prior to sample analysis. Since tumor cells are co-enriched with a high number of mononuclear cells subsequent immunocytochemical evaluation and detection of single tumor cells on microscopic slides is greatly limited. Likewise, genome analysis or molecular analysis employing nucleic acids as probes to hybridize with the specific biomarkers. For instance, RT-PCR or qRT-PCR is preferably employed. Upon enrichment of CTC the RNA can be analyzed. A standardized system for tumor cell enrichment is e.g. provided as OncoQuick® (Greiner Bio-One, Frickenhausen, Germany).

In specific aspects of the methods of the invention, the methods are non-invasive for solid tumor diagnosis, which in turn allow for diagnosis of a variety of conditions or diseases associated with solid tumor disease. In particular, the invention provides a non-invasive, non-surgical method.

The present invention is further illustrated by the following examples without being limited thereto.

### Example1: Identifying new candidate genes

The purpose of the present study was to find new candidate genes for the quantitative reverse transcriptipion PCR (qRT-PCR)-based detection of CTC in the peripheral blood of patients suffering from gynecological malignancies. We focused our interest in those genes that were (almost) not expressed in the peripheral blood of healthy females but appeared very highly expressed in tumor cells. To identify these genes, in the first phase of the project we compared the gene-expression signatures of various established breast, ovarian, cervical, and endometrial cancer cell lines to those of white blood cells from healthy donors using Applied Biosystems (AB) oligonucleotide microarrays. In the second phase of the project, we performed AB TaqMan® Low Density Array (TLDA) based qRT-PCR using microfluidic cards to verify the expression levels of a set of differentially expressed genes in the same cell lines, in primary tumor tissues, and finally in tumor cell-enriched blood samples taken from cancer patients as well as from healthy women processed equally as the patients' blood samples. Based on the results of these experiments, a panel of promising candidate genes was selected for future routine diagnosis of disseminated tumor cells.

### Methods

### Cell culture

10 breast cancer cell lines (MCF-7, T-47D, MDA-MB-231, Hs 578T, MDA-MB-435S, MDA-MB-453, BT-474, SK-BR-3, ZR-75-1, BT-549), 10 ovarian cancer cell lines (A2780, Caov-3, ES-2, NIHOVCAR-3, SK-OV-3, TOV-21G, TOV-112D, OV-90, OV-MZ-01 a, OV-MZ-6), 9 cervical cancer cell lines (HeLa, SW756, GH354, Ca Ski, C-4 I, C-33 A, HT-3, ME-180, SiHa) and 9 endometrial cancer cell lines (KLE, RL95-2, AN3 CA, HEC-1-B, Ishikawa, Colo 684, HEC-50-B, EN, EJ) were cultivated according to the recommended protocols. Cell lines were purchased from the American Type Culture Collection (ATCC, http://www.atcc.org) or from the European Collection of Cell Cultures (ECACC, http://www.ecacc.org.uk). The tumor cell lines EN and EJ were kindly provided by Keiichi Isaka (Department of Obstetrics and Gynecology at the Tokyo Medical University, Japan), OV-MZ-01 a and OV-MZ-6 by Volker Möbus (Department of Obstetrics and Gynecology, University of Ulm, D), and finally HEC-50-B was provided by Hiroyuki Kurarmoto (Department of Clinical Cytology Graduate School of Medical Sciences, School of Medicine, Kitasato University, Sagamihara, Kanagawa, Japan). The cells were harvested on at least three consecutive days and resuspended in lysis solution (Total RNA Isolation Mini Kit, Agilent Technologies, Waldbronn, Germany). The lysates were stored at -20°C prior to RNA extraction.

### Patients and healthy donors

From 2001 to 2006 peripheral blood samples were taken from 884 patients with benign or malign gynecological diseases in the Department of Obstetrics and Gynaecology and in the Department of Medicine I, Division of Oncology, and from 58 female healthy volunteers in the University Clinic for Blood Group Serology and Transfusion Medicine, Clinical Department for Transfusion Medicine, in the Department of Obstetrics and Gynaecology (all located at the MUW, Medical University of Vienna, Austria) and in ViennaLab (Vienna, Austria). The blood samples were collected in EDTA tubes and processed within 2 hours after venipuncture. For this study, we exluded patients with benign gynecological tumors, tumors of low malignant potential (i.e. borderline tumor of the ovaries), malignant tumors other than from the breast, the ovaries or the uterus, secondary malignoma, transplanted patients, and pregnant patients. Finally, 125 samples taken from patients with primary breast (N=21), ovarian (N=23), cervical and endometrial cancer (each 25 patients) before undergoing treatment (excision of the primary tumor or administration of a neoadjuvant chemotherapy), and from patients with advanced breast cancer (N=31) were included into the study.

In the same time period, fresh frozen tissue samples of patients with breast, ovarian, endometrial or cervical carcinoma were kindly provided by the Department of Gynecopathology, Clinical Institute for Pathology (MUW). Ovarian cancer tissues were partly collected by the Department of Obstetrics and Gynecology at the Charité-Universitatsmedizin Berlin, Germany. All tissue samples were stored in liquid nitrogen prior to homogenization.

The study inclusion criteria were the same as for blood samples; furthermore, recurrent patients and tissue samples taken after neoadjuvant chemotherapy were excluded. From a total of about 340 tumor tissues 50, 51 and 25 samples from patients with primary breast, ovarian or endometrial cancer, respectively were enrolled in the study. All peripheral blood and tumor tissue samples were collected with the patients' given written consent.

### Cell spiking

For sensitivity assays, a defined number of T-47D (American Type Culture Collection (ATCC, http://www.atcc.org)) breast cancer cells ranging from 4 to 4000 cells was added to each 15 ml pre-cooled peripheral venous blood taken from a healthy female donor in the Austrian National Red Cross Society. The negative control was unspiked blood from the same donor. Each blood sample was spiked in duplicates. After enrichment with OncoQuick (Greiner Bio-One, Frickenhausen, D) as per the manufacturer's instructions and resuspension in RLT-buffer (Qiagen RNA Isolation Kit), the corresponding lysates were pooled to compensate for varying recovery rates of the enrichment procedure. 1/6 of the extracted total RNA (Qiagen RNA Isolation Kit) was pre-amplified in triplicate reactions employing the TargetAmp™ 1-Round aRNA Amplification Kit (Epicentre, Madison WI, USA) as per the technical instructions. The pre-amplified RNA was converted into cDNA with M-MLV Reverse Transcriptase, RNase H Minus (Promega, Madison WI, USA) and random hexamers as primers. To assess the sensitivity of the TLDA platform to detect circulating tumor cells, qRT-PCR was performed using the TLDA format 96a as described below.

### Sample processing

For the comparative gene expression microarray studies the peripheral blood mononucleated cells (PBMC) were isolated from 50 ml blood donated by healthy females by a density gradient using Ficoll-Paque™ Plus (GE Healthcare Bio-Sciences AB, Uppsala, Sweden) as per the standard procedure. For gene expression analysis with qRT-PCR 15-25 ml peripheral blood taken from both healthy females and tumor patients was enriched for mononucleated cells using OncoQuick tubes (Greiner Bio-One, Frickenhausen, Germany) according to the manufacturer's instructions. The enriched cells were resuspended in the appropriate lysis solution.

Each 100 mg fresh frozen tumor tissue was ground for 2 min at 2000 rpm using a dismembrator (B. Braun Biotech., Melsungen, Germany) and further homogenized in lysis solution by intense vortexing.

All lysates were stored at -20°C prior to RNA extraction.

### RNA extraction

Total RNA was extracted with two commercially available kits depending on the amount of cells in the starting material: First, the Total RNA Isolation Mini Kit (Agilent Technologies, Waldbronn, Germany) was used for RNA extraction from cultivated tumor cells, from homogenized tumor tissue and from PBMC enriched by Ficoll-Paque™ Plus density gradient centrifugation. Total RNA samples were spectrophotometrically quantified and examined for residual genomic DNA by PCR employing primers which span exon 9 of breast cancer 2, early onset gene *BRCA2* (sense primer: 5'-ATA ACT GAA ATC ACC AAA AGT G-3' [SEQ ID No. 1]; antisense primer: 5'-CTG TAG TTC AAC TAA ACA GAG G-3' [SEQ ID No. 2]). Residual genomic DNA was digested by DNase I. Finally, quality assessment of the cell line-and PBMC-RNA was performed with RNA 6000 Nano LabChip Kit run on the 2100 bioanalyzer (Agilent Technologies, Waldbronn, Germany) and of RNA samples isolated from tumor tissues with denaturing agarose gel electrophoresis. The total RNAs extracted from at least three consecutive cell line harvests were combined to compensate for expression variations due to possibly varying culture conditions. Each the RNA pools and the RNA samples extracted from healthy PBMC were precipitated to reach a minimal final concentration of 1.5 µg/µl. Second, the RNeasy Micro Kit (Qiagen, Hilden, Germany) was used for RNA extraction from cells enriched by Oncoquick gradient. Because RNA yields were supposed to be low, we restrained from loosing further material by assessing the RNA quality or quantity in these samples.

### Expression profiling on Human Genome Microarrays

A total of 48 Human Genome Survey Microarrays Hs.v1 (Applied Biosystems, Foster City CA, USA) were run for comparing the gene expression signatures of 38 tumor cell lines (10 breast, 10 ovarian, 9 endometrial and 9 cervical cell lines) and 10 healthy control samples at GeneSys Laboratories GmbH (Muenster, Germany) using kits, reagents and the chemiluminescent microarray analyzer 1700 from AB according to the manufacturer's protocols. In brief, 20 µg total RNA was used to prepare digoxigenin-labeled cDNA, which developed a chemiluminescent signal after hybridizing to the 60-mer oligonucleotide probes spotted onto the microarray platform. Following to primary analysis and quality control using the AB Navigator Software Version 1.0.0.3 and to background correction, the data was normalized using the AB 1700 chemiluminescent microarray analyzer first by feature, then by spatial effects in the slide. Finally, a global normalization per slide was performed. Microarray expression measurements with a signal-to-noise ratio ≤3 and with flags >5000 were filtered out. No further normalization was applied. Genes with an average assay normalized signal (ANS) across the healthy control samples smaller than 1.5 were subjected to the maxT test on logged expression values from the R "multtest" package specifying a familywise error rate of 0.05 and 10000 permutations to find genes differentially expressed in any of the tumor cell lines compared to healthy control samples. Additionally we used a 50% one-sided trimmed maxT-test with a familywise error rate of 0.05 and 1000 permutations. This test only uses data values above the median in each group, and then compares the trimmed grouped means. Trimming is applied in each permutation. Therefore the 50% one-sided trimmed maxT-test will identify genes which are over-expressed in only a subgroup of the tumor cell lines.

Finally, from the resulting significant genes with a differential expression greater than 10 in the tumor cell lines compared to the healthy control samples 356 genes were selected for confirmatory gene expression profiling by qRT-PCR using the AB TaqMan® Low Density Array (TLDA) platform. Additionally the selected 356 genes were supplemented with 15 known or supposed markers for CTC detection.

### Verification of microarray results

The expression levels of the 356 genes selected from the microarray analyses and of the 15 known or supposed CTC markers were verified with qRT-PCR in a subset of each five breast, ovarian and endometrial cancer cell lines and in blood samples of 19 healthy females following enrichment with Oncoquick and RNA amplification as described below. qRT-PCR was performed using TLDA format 384 for the analysis of 380 gene targets in single reactions and of one mandatory endogenous control gene (glyceraldehyde-3-phosphate dehydrogenase [*GAPDH*]) in a quadruplicate reaction. The 380 gene targets consisted of additional 3 TaqMan® Endogenous Controls (beta-2-microglobulin [*B2M*], TAT-box binding protein [*TBP*]*,* and phosphoglyceratekinase 1 [PGK]) and 377 TaqMan® Gene Expression Assays specific for the 15 known or supposed CTC marker and specific for the previously selected differentially expressed genes according to a mapping of microarray probe IDs to assay IDs provided by AB. The RNA extracted from tumor cell lines was converted into cDNA with M-MLV Reverse Transcriptase, RNase H Minus (Promega, Madison WI, USA) and random hexamers as primers. The RNA extracted from healthy female PBMC was amplified following a modified version of a protocol published by Klein et al. (Nat Biotechnol, 2002. 20(4): p. 387-92). In short, the RNA was first converted into cDNA with M-MLV Reverse Transcriptase, RNase H Minus (Promega, Madison WI, USA) and random primers containing a 5'-oligo-dC flanking region (5'-[CCC]₅ TGC AGG N₆-3' [SEQ ID No. 3]; VBC Genomics, Vienna, Austria). Then, after generating a 3'-oligo-dG flanking region, the flanked cDNA was primed with CP2 (5'-TCA GAA TTC ATG [CCC]₅-3' [SEQ ID No. 4]; VBC Genomics) and amplified with Super Taq (HT Biotechnology Ltd.,Cambridge, Great Britain). The TLDA were loaded with the sample-specific PCR mix containing the template cDNA as recommended by the manufacturer (2 ng per well). The qRT-PCR amplifications were performed on the AB 7900HT Fast Real-time PCR System as per the technical instructions. Raw data were analyzed with the AB 7900 Sequence Detection Software version 2.2.2 using automatic baseline correction and manual cycle threshold (Ct) setting. Resulting Ct data was exported for further analysis. For downsizing the number of potential candidate genes from initially more than 30000 genes to about 100 genes, all genes with expression levels beyond the qRT-PCR detection limit (i.e. Ct 50) in the healthy control samples were excluded. The remaining genes were sorted by their arithmetic average Ct value of the 15 tumor cell lines in descending order. The first 93 genes were selected for qRT-PCR analysis of blood and tissue samples taken from tumor patients using the TLDA 96a format. Additionally, three genes (*B2M, GAPDH* and *PGK*) were selected as internal reference genes.

### Gene expression analysis of patients' blood and tissue samples

First, the expression of the previously selected 93 genes was measured in tumor tissue samples of patients with primary breast (N=50), ovarian (N=51) and endometrial cancer (N=25) with qRT-PCR using the TLDA 96a format to verify the adequacy for their intended use as candidate markers for the detection of CTC in the blood of cancer patients. Then, using the same qRT-PCR platform, the gene expression was evaluated in blood samples of healthy female volunteers (N=26) and in peripheral blood samples of tumor patients with primary breast (N=21), ovarian (N=23), cervical and endometrial cancer (each 25 patients), and with advanced breast cancer (N=31) following enrichment with OncoQuick density gradient centrifugation and RNA amplification as described below. For the gene expression analysis of tumor tissues, RNA was converted into cDNA by Omniscript Reverse Transcriptase (Quiagen, Hilden, D) using an oligo-dT-flanked primer. For the gene expression analysis blood samples, 1/6 of the total RNA amount was amplified employing the TargetAmp™ 1-Round aRNA Amplification Kit (Epicentre, Madison WI, USA) as per the technical instructions. The amplified RNA was converted into cDNA with M-MLV Reverse Transcriptase, RNase H Minus (Promega, Madison WI, USA) and random hexamers as primers. Loading the microfluidic cards, qRT-PCR amplification, and raw data analysis were performed as described in the last preceding paragraph. All samples were analyzed as duplicates. The mean of the resulting duplicate Ct values was used as a quantitative value. If only one of the duplicates was positive (i.e. Ct<50), the one Ct value was taken. Low-level expression of many genes in the peripheral blood of the healthy control group decreased the overall assay specificity and required the introduction of a cut-off threshold value to separate the tumor patients group from the healthy control group:

A threshold value Tₓ for each gene X was set to three standard deviations from the mean dCtₓ value in the control group. dCtₓ values were calculated by normalizing the average expression of gene X to the average expression of the endogenous control gene *GAPDH.* If only one healthy control sample revealed detectable gene expression, the one dCtₓ was taken as cut-off threshold value. A tumor patient was considered to be positive for the molecular analysis of gene X, if dCtₓ was below the defined threshold value Tₓ.

Additionally, we performed human mammaglobin-specific qRT-PCR of the same set of breast cancer blood samples and of a further set of healthy female controls after cell enrichment and RNA preamplification as described above. Mammaglobin expression was analyzed in duplicate reactions using individual AB TaqMan® Pre-Developed Assay Reagents (Hs00267190_m1)

**Table 1a -**

| ***GENE*** | ***PRIMER*** | ***PRIMER SEQUENCE*** |
|---|---|---|
| FN1 | FN1 sense primer | 5'-agg aaa cct gct cca gtg cat-3' SEQ ID No. 5 |
| FN1 | FN1 antisense primer | 5'-cgg ttg gta aac agc tgc acg-3' SEQ ID No. 6 |
| FN1 | FN1 probe | 5'-aca tcg agc gga tct ggc ccc-3' SEQ ID No. 7 |
| RBPMS | RBPMS sense primer | 5'-caa acc tcg gga gct cta tct g-3' SEQ ID No. 8 |
| RBPMS | RBPMS antisense primer | 5'-cta cag gct gtt tag atg tga gct tta t-3' SEQ ID No. 9 |
| RBPMS | RBPMS probe | 5'-ttt tca gac cat tta agg gct atg agg gtt ctc-3' SEQ ID No. 10 |
| TM4SF1 | TM4SF1 sense primer | 5'-ccg ctt cgt gtg gtt ctt tt-3' SEQ ID No. 11 |
| TM4SF1 | TM4SF1 antisense primer | 5'-cag ccc aat gaa gac aaa tgc-3' SEQ ID No. 12 |
| TM4SF1 | TM4SF1 probe | 5'-agg tgg cct gct gat gct cct gc-3' SEQ ID No. 13 |

**Table 1b -**

| ***GENE*** | ***PRODUCT NUMBER (Applied Biosystems** -* ***TaqMan*® *Gene Expression Assays)*** |
|---|---|
| MAL2 | MAL2-Hs00294541_m1 |
| CCNE2 | CCNE2-Hs00372959_m1 |
| EMP2 | EMP2-Hs00171315_m1 |
| PPIC | PPIC-Hs00181460_m1 |
| TFF1 | TFF1-Hs00170216_m1 |
| DKFZp762E1312 | DKFZp762E1312 Hs00251144_m1 |
| SLC6A8 | SLC6A8 Hs00373917_g1 |
| S100A16 | S100A16 Hs00293488_m1 |
| AGR2 | AGR2 Hs00180702_m1 |
| FXYD3 | FXYD3-Hs00254211_m1 |
| EpCAM | EpCAM-Hs00158980_m1 |
| Mammaglobin | Mammaglobin-Hs00267190_m1 |

### Results

### RNA quality assessment

Prior to microarray hybridization and qRT-PCR analysis, the RNA extracted from the tumor cell lines and the healthy PBMC was checked for quality with the RNA 6000 Nano LabChip Kit run on the Agilent 2100 bioanalyzer. The Agilent 2100 expert software provides an algorithm to calculate the RNA Integrity Number (RIN), which classifies the RNA quality based on a numbering system from 1 to 10, with 1 being the most degraded profile and 10 being the most intact. As a result, 85% of the RNA samples had a very good RNA quality (RIN≥8) and 60% an even excellent quality indicated by a RIN≥9 (see Table 1).

### Table 2 - RNA quality of microarray samples

Quality of RNA samples isolated from cancer cell lines and from PBMC of healthy female donors was assessed prior to microarray analysis. The RNA Integrity Number (RIN) calculated by the Agilent RNA 6000 Nano LabChip Kit software is given for all breast, cervical, endometrial and ovarian cancer cell lines and for the healthy control samples analysed with the AB microarrays (N/A; the software failed to calculate the RIN).

| **CANCER CELL LINES** | | | | | | | |
|---|---|---|---|---|---|---|---|
| **Breast** | | **Cervical** | | **Endometrial** | | **Ovarian** | |
| BT474 | 6.7 | C-33 A | 9.0 | AN3 CA | 10.0 | A2780 | 9.2 |
| BT-549 | 9.9 | C-4 I | 9.9 | Colo 684 | 8.9 | Caov-3 | 9.1 |
| Hs 578T | 10.0 | Ca Ski | 9.7 | EJ | 10.0 | ES-2 | 10.0 |
| MCF-7 | N/A | GH354 | 9.8 | EN | 9.8 | NIHOVCAR-3 | N/A |
| MDA-MB-231 | 8.0 | HeLa | 9.5 | HEC-1-B | 7.0 | OV-90 | 10.0 |
| MDA-MB-435s | 8.8 | HT-3 | 10.0 | HEC-50-B | 9.9 | OV-MZ-01a | 9.1 |
| MDA-MB-453 | 8.2 | ME-180 | 9.7 | Ishikawa | 7.5 | OV-MZ-6 | 9.1 |
| SK-BR-3 | N/A | SiHa | 10.0 | KLE | 10.0 | SK-OV-3 | 8.6 |
| T-47D | 9.9 | SW756 | 10.0 | RL95-2 | 9.8 | TOV-112D | 9.2 |
| ZR-75-1 | 9.3 | | | | | TOV-21 G | 8.8 |

| **HEALTHY CONTROL SAMPLES** | | | | | | | |
|---|---|---|---|---|---|---|---|
| S211 | 8.4 | S210 | 9.2 | S208 | 8.1 | S217 | 7.1 |
| S203 | 8.6 | S212 | 8.7 | S209 | 9.3 | S218 | 7.9 |
| S204 | 8.5 | S213 | 8.3 | S205 | 7.2 | S216 | 6.8 |

### Differentially expressed genes in tumor cell lines compared to healthy PBMC

We compared the gene expression profile of 38 established gynecological cancer cell lines to those of PBMC taken from 10 healthy donors using Applied Biosystems Human Genome Survey Microarrays Hs.v1 to identify genes that were (almost) not expressed in the peripheral blood of healthy females but appeared very highly expressed in the tumor cell lines. From the 18151 (54.8%) genes with an average ANS<1.5 across the ten PBMC control samples maxT-test identified 518, 575, 541, and 537 genes differentially expressed in the breast, cervical, endometrial and ovarian cancer cell lines, respectively, compared to the healthy controls, comprising 66, 61, 87 and 61 genes with tumor site specific expression for the respective cancer cell lines. The 50% one-sided trimmed maxT-test identified further 25, 27, 20 and 29 genes in breast, cervical, endometrial and ovarian cancer cell lines differentially expressed compared to the healthy controls. Finally, 356 differentially expressed genes were chosen for confirmatory gene expression profiling with qRT-PCR using the TLDA 384 format, consisting of 337 genes identified by maxT-test and 19 by 50% one-sided trimmed maxT-test, and comprising 4 genes represented with more than one TaqMan® Assay (*EFEMP1, EPS8L1, CRYZL1* and *PCDHG).* Additionally we decided to analyze 9 published tumor markers (*ERBB2*, *ESR1, PGR, PLAT, SCGB2A1, SCGB2A2, SERPINE1, SERPINE2* and *TFF1*) and 6 candidates for CTC detection as described in WO2006018290A2. (*COL3A1, GHR, CALB1, LPHN1, FN1* and *EDNRA)* with qRT-PCR using the TLDA 384 format.

### Verification of microarray results

The expression levels of the 356 genes selected from the microarray analyses and of the 15 known or supposed CTC marker were verified with qRT-PCR in blood samples of 19 healthy females compared to each 5 breast, ovarian and endometrial cancer cell lines using the TLDA 384 format. As a result, the expression levels of 146 genes were below the detection limit of qRT-PCR (i.e. Ct 50) in the healthy controls. Therefore, these genes were identified as potential markers for the detection of circulating tumor cells in the blood of cancer patients, in principle. They were sorted by their arithmetic average Ct value of the 15 tumor cell lines in descending order and the first 93 genes were selected for further gene expression analysis of patients' samples using the TLDA 96a format (see Table 2). None of the 15 known or supposed markers for CTC detection was considered for further investigations either due to detectable expression levels (*ERBB2, ESR1, SERPINE1, SERPINE2* and *FN1*) in healthy controls or due to gene expression in only part of the tumor cell lines.

### Table 3 - Gene identifiers of the TLDA 96a platform

For qRT-PCR analysis of blood and tumor tissue samples taken from cancer patients 93 genes were selected as promising candidate genes for CTC detection. Additionally, 3 house-keeping genes *(B2M, GAPDH, PGK1*) were chosen as internal reference.

| GENE ID | GENE SYMBOL | GENE NAME |
|---|---|---|
| hCG1640825 | *ALDH1B1* | aldehyde dehydrogenase 1 family, member B1 |
| hCG14791 | *AMOTL2* | angiomotin like 2 |
| hCG1646237 | *ANKRD9* | ankyrin repeat domain 9 |
| hCG1810762 | *ANTXR1* | anthrax toxin receptor 1 |
| hCG16103 | *ARK5* | NUAK family, SNF1-like kinase, 1 |
| hCG2039667 | *ASPM* | asp (abnormal spindle)-like, microcephaly associated (Drosophila) |
| hCG31281 | *AURKB* | aurora kinase B |
| hCG1786707 | *B2M* | Beta-2-microglobulin |
| hCG25202 | *B4GALT2* | UDP-Gal:betaGlcNAc beta 1,4-galactosyltransferase, polypeptide 2 |
| hCG2028796 | *BCAR3* | breast cancer anti-estrogen resistance 3 |
| hCG2032093 | *BCLP* | transmembrane protein 54 |
| hCG38062 | *C20orf129* | chromosome 20 open reading frame 129 |
| hCG17453 | *CALD1* | caldesmon 1 |
| hCG37307 | *CAP2* | CAP, adenylate cyclase-associated protein, 2 (yeast) |
| hCG15054 | *CCNE2* | cyclin E2 |
| hCG23164 | *CDC20* | CDC20 cell division cycle 20 homolog (S. cerevisiae) |
| hCG17896 | *CDC45L* | CDC45 cell division cycle 45-like (S. cerevisiae) |
| hCG23394 | *CDCA5* | cell division cycle associated 5 |
| hCG2013407 | *CHPF* | unassigned |
| hCG32502 | *CT120* | family with sequence similarity 57, member A |
| hCG23652 | *CYR61* | cysteine-rich, angiogenic inducer, 61 |
| hCG20139 | *DCBLD2* | discoidin, CUB and LCCL domain containing 2 |
| hCG40384 | *DEPDC1B* | DEP domain containing 1 B |
| hCG2012788 | *DKFZp762E1312* | hypothetical protein DKFZp762E1312 |
| hCG14644 | *EMP2* | epithelial membrane protein 2 |
| hCG1640408 | *ENAH* | enabled homolog (Drosophila) |
| hCG1785709 | *EPB41L1* | erythrocyte membrane protein band 4.1-like 1 |
| hCG1983413 | *EPPB9* | B9 protein |
| hCG1778932 | *ESPL1* | extra spindle poles like 1 (S. cerevisiae) |
| hCG32848 | *EXTL2* | exostoses (multiple)-like 2 |
| hCG1811328 | *FARP1* | FERM, RhoGEF (ARHGEF) and pleckstrin domain protein 1 (chondrocyte-derived) |
| hCG16250 | *FAT* | FAT tumor suppressor homolog 1 (Drosophila) |
| hCG15599 | *FBLN1* | fibulin 1 |
| hCG40645 | *FLJ11196* | La ribonucleoprotein domain family, member 6 |
| hCG25681 | *FLJ31434* | mannosidase, endo-alpha-like |
| hCG1731745 | *FOXM1* | forkhead box M1 |
| hCG2005673 | *GAPDH* | glyceraldehyde-3-phosphate dehydrogenase |
| hCG39145 | *GNA*/*1* | guanine nucleotide binding protein (G protein), alpha inhibiting activity polypeptide 1 |
| hCG27693 | *GPCR5A* | G protein-coupled receptor, family C, group 5, member A |
| hCG23322 | *GPT2* | glutamic pyruvate transaminase (alanine aminotransferase) 2 |
| hCG1984823 | *GTF2IRD1* | GTF2I repeat domain containing 1 |
| hCG1992685 | *GTSE1* | G-2 and S-phase expressed 1 |
| hCG33002 | *HIG2* | hypoxia-inducible protein 2 |
| hCG29840 | *HUMPPA* | paraneoplastic antigen |
| hCG29787 | *KDELC1* | KDEL (Lys-Asp-Glu-Leu) containing 1 |
| hCG2010805 | *KDELR3* | KDEL (Lys-Asp-Glu-Leu) endoplasmic reticulum protein retention receptor 3 |
| hCG1776116 | *KIF2C* | kinesin family member 2C |
| hCG17112 | *LAMB1* | laminin, beta 1 |
| hCG31813 | *LOC116238* | hypothetical protein BC014072 |
| hCG1653390 | *MAL2* | mal, T-cell differentiation protein 2 |
| hCG39301 | *MEIS2* | Meis1, myeloid ecotropic viral integration site 1 homolog 2 (mouse) |
| hCG1811944 | *MID1* | midline 1 (Opitz/BBB syndrome) |
| hCG38470 | *MYBL2* | v-myb myeloblastosis viral oncogene homolog (avian)-like 2 |
| hCG28204 | *NQO1* | NAD(P)H dehydrogenase, quinone 1 |
| hCG21309 | *NR2F2* | nuclear receptor subfamily 2, group F, member 2 |
| hCG1642334 | *OIP5* | Opa interacting protein 5 |
| hCG32369 | *ORC6L* | origin recognition complex, subunit 6 homolog-like (yeast) |
| hCG25176 | *PACSIN3* | protein kinase C and casein kinase substrate in neurons 3 |
| hCG23646 | *PARVA* | parvin, alpha |
| hCG1982215 | *PCDHGC3* | unassigned |
| hCG20034 | *PGK1* | phosphoglycerate kinase 1 |
| hCG40225 | *PHLDB1* | pleckstrin homology-like domain, family B, member 1 |
| hCG15614 | *PKMYT1* | protein kinase, membrane associated tyrosine/threonine 1 |
| hCG17154 | *PLAT* | plasminogen activator, tissue |
| hCG20966 | *PLEKHC1* | pleckstrin homology domain containing, family C (with FERM domain) member 1 |
| hCG39584 | *PLK2* | Polo-like kinase 2 (Drosophila) |
| hCG18528 | *PPAP2C* | phosphatidic acid phosphatase type 2C |
| hCG37361 | *PPIC* | peptidylprolyl isomerase C (cyclophilin C) |
| hCG1811513 | *PSPH* | phosphoserine phosphatase |
| hCG25208 | *PTPRF* | protein tyrosine phosphatase, receptor type, F |
| hCG38803 | *PYCR1* | pyrroline-5-carboxylate reductase 1 |
| hCG1998805 | *RAD51* | RAD51 homolog (RecA homolog, E. coli) (S. cerevisiae) |
| hCG1768049 | *RAI14* | retinoic acid induced 14 |
| hCG1818529 | *RAMP* | denticleless homolog (Drosophila) |
| hCG2010626 | *RBM9* | RNA binding motif protein 9 |
| hCG1982350 | *RHPN2* | rhophilin, Rho GTPase binding protein 2 |
| hCG1743779 | *S100A16* | S100 calcium binding protein A16 |
| hCG15745 | *SDC2* | syndecan 2 (heparan sulfate proteoglycan 1, cell surface-associated, fibroglycan) |
| hCG20638 | *SGCB* | sarcoglycan, beta (43kDa dystrophin-associated glycoprotein) |
| hCG30092 | *SHB* | Src homology 2 domain containing adaptor protein B |
| hCG2007960 | *SLC6A8* | unassigned |
| hCG1980650 | *SMARCA1* | SWI/SNF related, matrix associated, actin dependent regulator of chromatin, subfamily a, member 1 |
| hCG41293 | *SMTN* | smoothelin |
| hCG31796 | *SPAG5* | sperm associated antigen 5 |
| hCG17043 | *Spc25* | spindle pole body component 25 homolog (S. cerevisiae) |
| hCG39506 | *SPR* | sepiapterin reductase (7,8-dihydrobiopterin:NADP+ oxidoreductase) |
| hCG1820982 | *SPRY4* | sprouty homolog 4 (Drosophila) |
| hCG39095 | *STK6* | serine/threonine kinase 6 |
| hCG29392 | *TK1* | thymidine kinase 1, soluble |
| hCG19468 | *TM4SF6* | tetraspanin 6 |
| hCG32771 | *TOM1L1* | target of myb1-like 1 (chicken) |
| hCG20940 | *TPD52L1* | tumor protein D52-like 1 |
| hCG2019820 | *TRIB3* | tribbles homolog 3 (Drosophila) |
| hCG41040 | *WDR34* | WD repeat domain 34 |
| hCG21216 | *WWTR1* | WW domain containing transcription regulator 1 |
| hCG1645136 | *ZDHHC9* | zinc finger, DHHC-type containing 9 |

### Cell spiking

To assess the applicability of the TLDA platform for the qRT-PCR based detection of circulating tumor cells, the expression levels of the specified 96 genes were measured in healthy female blood samples spiked with T-47D breast cancer cells. As a result, *CCNE2* and *MAL2* transcripts were not detected in the unspiked blood, but in blood samples spiked with at least 26 and 2.6 tumor cells per ml blood, respectively. Expression levels of (*EMP2, PPIC, DKFZp762E1312*, and *SLC6A8*) were measured in the unspiked blood beyond the detection limit of PCR (i.e. Ct 50), but decreasing Ct values were observed proportionally to the increasing number of added tumor cells. A minimum decrease by 3 Ct values compared to the unspiked blood was observed when at least 2.6 (*EMP2*, *PPIC*) and 26 tumor cells per ml blood (*DKFZp762E1312, SLC6A8*) were added to 1 ml blood. Furthermore, the spiking experiments revealed that qRT-PCR might be less sensitive using the TLDA platform than using conventional PCR tubes, because linear amplification patterns distinguishing each 10-fold dilution were only observed with Ct values smaller than 35.

### Gene expression analysis of patients' blood and tissue samples

First, the expression levels of the previously selected 93 genes were measured in tumor samples of patients with primary breast (N=50), ovarian (N=51) and endometrial cancer (N=25) to verify the adequacy for their intended use as candidate markers for the detection of circulating tumor cells in the blood of cancer patients. The qRT-PCR analyis using TLDA revealed that mRNA transcripts were detected in the tumor tissues, at least in some of the patients, indicating that any of the 93 genes might be an appropriate CTC marker. We observed that the house-keeping gene expression levels were lower in ovarian cancer tissues than in tumor tissues of breast and endometrial cancer patients *(GAPDH* 24.2±2.6, 22.2±1.2, 22.7±1.4 (SD) Ct; *B2M* 22.1±3.4, 18.1±1.5, 17.7±1.9 (SD) Ct; PGK25.5±2.7, 23.5±1.1, 22.4±3.0 (SD) Ct in the respective tumor patients). None of the 93 genes turned out to be tumor-site specific except for two genes: *PLEKHC1* (pleckstrin homology domain containing, family C [with FERM domain] member 1) and *SGCB* (sarcoglycan beta) transcripts were detected in ovarian cancer patients only, although being detected in cancer cell lines of breast and endometrial origin either. Interestingly, expression of the selected 93 genes was detected in more ovarian cancer patients than in breast and endometrial cancer patients (median percentage of positive patients in the respective tumor groups was 78.4%, 64.0% and 32.0%).

Furthermore, the expression of the previously selected 93 genes was evaluated in an additional set of blood samples from 26 healthy female volunteers (N=26), in pretreatment blood samples from primary breast (N=21), ovarian (N=23), cervical and endometrial cancer (each N=25) patients, and in blood samples from patients with advanced breast cancer (N=31) after Oncoquick enrichment and RNA amplification. Low-level expression of many genes in the peripheral blood of the healthy control group due to a more efficient RNA amplification than applied in preliminary gene expression analysis of tumor cell lines and healthy controls decreased the overall assay specificity and required the introduction of a cut-off threshold value to separate the tumor patients and the healthy control group. We found out, that at a threshold of three standard deviations from the mean expression level of the healthy controls, each 17 (68.0%) cervical and endometrial cancer, 6 (26.1 %) ovarian cancer and 8 (38.1 %) primary breast cancer patients were positive for at least one out of 93 potential candidate genes. At the same threshold, 27 (87.1%) patients with advanced breast cancer were positive for at least one gene. From all candidate genes, only 40 were able to identify patients at the defined respective threshold. From these genes, 33 and 15 identifed advanced and primary breast cancer patients, respectively, each 14 identified cervical and endometrial cancer patients and 4 genes identified ovarian cancer patients. The remaining 55 genes were not informative at all due to similar expression levels in both the healthy control group and any of the tumor groups.

On an individual marker basis, at the defined threshold 7 genes were over-expressed in at least 10% of the tumor patients as follows:
*CCNE2* (cyclin E2) in 40.0% of the patients with cervical cancer, in 36.0% of the patients with endometrial cancer, 13.0% of the patients with ovarian cancer, 23.8% of the patients with primary breast cancer and in 32.3% of the patients with advanced breast cancer, *GTF2IRD1* (GTF2I repeat domain containing protein 1) in 28.0% of the patients with endometrial cancer and in 16.0% of the patients with cervical cancer, *MAL2* (Mal, T-cell differentiation protein 2) in 20% of the patients with endometrial cancer and in 19.4% of the patients with advanced breast cancer, *EMP2* (epithelial membrane protein 2) in 12% of the patients with endometrial cancer and in 32.3% of the patients with advanced breast cancer, *SLC6A8* (solute carrier family 6 [neurotransmitter transporter, creatine], member 8) in 45.2% of the patients with advanced breast cancer and in 12% of the patients with endometrial cancer, *DKFZp762E1312* (hypothetical protein DKFZp762E1312) in 25.8% of the patients with advanced breast cancer and *PPIC* (peptidyl-prolyl-isomerase C) in 19.4% of the patients with advanced breast cancer.

Additionally, human mammaglobin A-specific qRT-PCR of the same set of breast cancer blood samples and of a further set of healthy female controls confirmed the published tissue specific expression of mammaglobin. Transcripts were detected in 38.7% of the advanced, but in neither the primary breast cancer patients nor the healthy controls.

To increase the detection sensitivity of circulating tumor cells we intended to identify a panel of genes for future multi-marker qRT-PCR based analysis of peripheral blood samples obtained from cancer patients. For this purpose we selected genes prevalently over-expressed in metastatic patients, as the occurrence of circulating tumor cells is most likely in advanced disease. We supposed that from the combined analysis of the six qRT-PCR positive genes in more than 10% of the patients with advanced breast cancer (*CCNE2, DKFZp762E1312, EMP2*, *MAL2, PPIC* and *SLC6A8*), 81% of the patients with advanced and 29% of the patients with primary breast cancer would be positive for at least one of the six genes. In the cervical, endometrial and ovarian group, the ratio of positive patients would be 44%, 64% and 19%, respectively.

### Discussion

Using a stepwise approach which combined genome-wide expression profiling and TaqMan® based qRT-PCR we surprisingly identified *CCNE2* alone or in a multimarker panel of and six genes (*CCNE2, DKFZp762E1312*, *EMP2*, *MAL2, PPIC*, and *SLC6A8*) as potential markers for the detection of circulating tumor cells in the peripheral blood of patients with gynecological malignancies. Although implicated with cancer, these genes have not previously been specified for the detection of circulating tumor cells in cancer patients at least to our knowledge. Evidence that the genes mentioned above might be promising targets for CTC detection is that more patients with advanced than with newly diagnosed breast cancer (81% vs. 29%) showed higher expression levels compared to healthy females. Interestingly, also patients with other gynecological malignancies (i.e. cervical, endometrial and ovarian cancer) would be positive in the combined PCR-based molecular analysis of these six genes. Gene expression was also analyzed in tissue samples of ovarian, breast, and endometrial cancer patients. We found that *CCNE2, DKFZp762E1312*, *EMP2*, and *SLC6A8* gene expression in tumor tissues reflects the tumor stage rather than the tumor location, as more ovarian cancer patients than breast or endometrial cancer patients were qRT-PCR positive. In ovarian cancer most of the patients (64.7%) presented with advanced disease at the time of the primary operation (tumor stage pT≥3), whereas in endometrial and breast cancer most patients were diagnosed with an early stage disease (76.0% pT1 and 90.0% pT1 or pT2, respectively). In contrast, *MAL2* and *PPIC* gene expression was detected in almost all patients irrespective of the tumor location. We suppose that the detection of *CCNE2* transcripts alone or preferably together with *MAL2* transcripts in the blood of cancer patients but not of healthy females is indicative for CTC presence, which had not been verified by immunocytochemistry. The observed increase of *CCNE2* mRNA levels in the diseased group compared to the healthy control group was surprising, since they are reported to be undetectable in normal quiescent cells arrested in G₀ (Lauper, N., et al., Oncogene, 1998. 17(20): p. 2637-43). CTC are, however, described as non-proliferative (Muller, V., et al. Clin Cancer Res, 2005. 11(10): p. 3678-85).

Interestingly, both *CCNE2* and *MAL2* are located on chromosome 8q, a region which is frequently increased in copy number in breast and other type of cancers; one of the most important target genes affected by gains and amplifications of 8q is the *MYC* oncogene. In contrast, *DKFZp762E1312, EMP2, PPIC,* and *SLC6A8* transcripts were also detected in the blood of healthy females. Applying a rigorous threshold level (three standard deviations from the mean expression in healthy female blood), each 17 (68.0%) cervical and endometrial cancer, 6 (26.1 %) ovarian cancer and 8 (38.1 %) primary breast cancer patients were positive for at least one out of 93 potential candidate genes. At the same threshold, 27 (87.1 %) patients with advanced breast cancer were positive for at least one gene. In spiking experiments the detection limit of TLDA-based qRT-PCR following tumor cell enrichment was 1 and 10 tumor cells per 2.5x10⁶ peripheral blood cells employing *MAL2-* and *CCNE2*-specific primers as used with the systems described above, respectively, which corresponds to a detection sensitivity of 2.6 and 26 tumor cells per ml whole blood.

### Conclusions

Our findings that the qRT-PCR-based multi-marker analysis of six genes more than doubled the percentage of advanced breast cancer patients positive compared to the analysis of mammaglobin alone, suggest that the up-regulation of these six genes in the blood indicates the presence of circulating tumor cells. This multi marker analysis might provide a tool for the early diagnosis, clinical monitoring and treatment control of gynecological malignancies, which is fast and simple to perform and easily tolerable for the patient.

### Example 2: Breast cancer study

The purpose of this study was to identify markers which could be used for diagnostic purposes in addition to CCNE2.

### Methods

### Sample processing

15-25 ml peripheral blood taken from both 26 healthy females and from 20 patients with advanced breast cancer was enriched for mononucleated cells using OncoQuick tubes (Greiner Bio-One, Frickenhausen, Germany) according to the manufacturer's instructions. The enriched cells were resuspended in RLT lysis solution (Qiagen, Hilden, Germany). All lysates were stored at -20°C prior to RNA extraction.

Total RNA was extracted with the RNeasy Micro Kit (Qiagen, Hilden, Germany). Because RNA yields were supposed to be low, we restrained from loosing further material by assessing the RNA quality or quantity. The RNA was converted into cDNA as follows: First, the total amount of extracted RNA was pre-incubated with 300 ng random hexamer at 65°C for 5 minutes. Then 200 U M-MLV Reverse Transcriptase, RNase H Minus, Point Mutant, M-MLV Reverse Transcriptase 1X Reaction Buffer, 10 U RNasin® Plus RNase Inhibitor (all purchased from Promega, Madison WI), 50 nmol of an equimolar mix of dATP, dTTP, dCTP and dGTP (Amersham Biosciences, Freiburg, Germany) and water was added to a final reaction volume of 20 µl. The reaction was performed at 55°C for 50 minutes after a pre-incubation step at 20°C for 10 min. Finally, the reaction was stopped by heating up to 94°C for 5 min.

### Quantitative reverse-transcription PCR (qRT-PCR)

Gene expression was analyzed in duplicate reactions using individual TaqMan® Pre-Developed Assay Reagents specific for *AGR2*, *S100A16*, *TFF1,* and *FXYD3*, consisting of two unlabeled PCR primers and one FAM™ dye-labeled TaqMan^{®} MGB probe as used with the systems described above. The total volume of the reactions was 14 µl containing 7 µl 2x TaqMan^{®} Universal PCR Master, 0.7 µl TaqMan^{®} Pre-developed Assay Reagents, and 4 µl fivefold diluted cDNA template. The PCR amplification was performed using the AB 7900HT Fast Real-time PCR System and consisted of an initial incubation at 50°C for 2 min., then 95°C for 10 min., followed by 50 cycles of denaturation at 95°C for 15 s and extension at 60°C for 1 min. The data were analyzed with the AB7900 Sequence Detection Software version 2.2.2 using automatic baseline correction and cycle threshold setting. Resulting cycle threshold (Ct) data was exported for further analysis. Consumables, equipment and software were purchased from Applied Biosystems, Foster City CA, USA.

All samples were analyzed as duplicates. The mean of the resulting duplicate Ct values was used as a quantitative value. If only one of the duplicates was positive (i.e. Ct<50), the one Ct value was taken. Low-level expression of *AGR2*, *S100A16* and *FXYD3* in the peripheral blood of the healthy control group required the introduction of a cut-off threshold value to separate the tumor patients group from the healthy control group:
A threshold value Tₓ for each gene X was set to three standard deviations from the average Ct_{X} value in the control group. If only one healthy control sample revealed detectable gene expression, the one Ct_{X} was taken as cut-off threshold value. A tumor patient was considered to be positive for the molecular analysis of gene X, if Ct_{X} was below the defined threshold value T_{X}.

### Results

Analyzing *AGR2*, *S100A16*, *TFF1*, and *FXYD3* mRNA levels in the blood of breast cancer patients with advanced disease we found overexpression of the respective genes in 10%, 25%, and each 20% of the patients analyzed compared to healthy females. We suppose that from the combined analysis of the above-mentioned genes 40% of the patients with advanced breast cancer would be positive for at least one of these genes.

### Example 3: Breast/ Ovary cancer study

### Methods

### Sample processing

15-25 ml peripheral blood taken from both 17 healthy females and from 84 cancer patients (primary breast cancer: N=21, advanced breast cancer: N=31, primary ovarian cancer: N=22, advanced ovarian cancer: N=10) was enriched for mononucleated cells using OncoQuick tubes (Greiner Bio-One, Frickenhausen, Germany) according to the manufacturer's instructions. The enriched cells were resuspended in RLT lysis solution (Qiagen, Hilden, Germany). All lysates were stored at -20°C prior to RNA extraction.

Total RNA was extracted with the RNeasy Micro Kit (Qiagen, Hilden, Germany). 1/6 of the total RNA amount was amplified employing the TargetAmpTM 1-Round aRNA Amplification Kit (Epicentre, Madison WI, USA) as per the technical instructions. The amplified RNA was converted into cDNA with M-MLV Reverse Transcriptase, RNase H Minus (Promega, Madison WI, USA) and random hexamers as primers.

### Quantitative reverse-transcription PCR (qRT-PCR)

Gene expression was analyzed in duplicate reactions using either TaqMan^{®} Pre-Developed Assay Reagents specific for *SCGB2A2* and *EPCAM* consisting of two unlabeled PCR primers and one FAM™ dye-labeled TaqMan^{®} MGB probe or individual primers and 5'-FAM™ dye-labeled probes (VBC-Biotech Services GmbH, Vienna, A) specific for *FN1*, *RBPMS* and *TM4SF1* as described above.

The total volume of the reactions was 14 µl containing 7 µl 2x TaqMan^{®} Universal PCR Master, 0.7 µl TaqMan^{®} Pre-developed Assay Reagents or the appropriate amount of individual primers and probes, and 4 µl fivefold diluted cDNA template. The PCR amplification was performed using the AB 7900HT Fast Real-time PCR System and consisted of an initial incubation at 50°C for 2 min., then 95°C for 10 min., followed by 50 cycles of denaturation at 95°C for 15 s and extension at 60°C for 1 min. The data were analyzed with the AB7900 Sequence Detection Software version 2.2.2 using automatic baseline correction and cycle threshold setting. Resulting cycle threshold (Ct) data was exported for further analysis. Consumables, equipment and software were purchased from Applied Biosystems, Foster City CA, USA.

All samples were analyzed as duplicates. The mean of the resulting duplicate Ct values was used as a quantitative value. If only one of the duplicates was positive (i.e. Ct<50), the one Ct value was taken. Low-level expression of all genes except of *SCGB2A2* in the peripheral blood of the healthy control group required the introduction of a cut-off threshold value to separate the tumor patients group from the healthy control group:
A threshold value T_{X} for each gene X was set to three standard deviations from the average dCt_{X} (gene expression normalized to *GAPDH* expression) value in the control group. If only one healthy control sample revealed detectable gene expression, the one dCt_{X} was taken as cut-off threshold value. A tumor patient was considered to be positive for the molecular analysis of gene X, if dCt_{X} was below the defined threshold value T_{X}.

### Results

Overexpression was found in patients with breast or ovarian cancer as follows:

| | Breast cancer | | Ovarian cancer | |
|---|---|---|---|---|
| | Primary | advanced | Primary | advanced |
| *SCGB2A2* | 0% | 34.4% | 0% | 0% |
| *EPCAM* | 0% | 6.5% | 0% | 0% |
| *FN1* | 5.3% | 6.5% | 4.5% | 0% |
| *TM4SF1* | 0% | 6.5% | 4.5% | 0% |
| *RBPMS* | 10.5% | 3.2% | 4.5% | 0% |

## Claims

1. Method of detecting CCNE2 in a body fluid sample of a patient for detecting susceptibility to breast, ovarian, endometrial, or cervical cancer, comprising
a. testing the CCNE2 nucleic acid expression in a tumor cell-enriched blood sample from said patient, and
b. comparing the result with a reference level, wherein an elevated level is an indication that the patient is at risk of a tumor disease, which is breast cancer, ovarian cancer, endometrial cancer, or cervical cancer.

2. Method according to claim 1, wherein a comparative gene expression analysis is performed.

3. Method according to any of claims 1 or 2, wherein at least one further marker selected from the group of DKFZp762E1312, EMP2, MAL2, PPIC, SLC6A8, GTF2IRD1, AGR2, FXYD3, S100A16, TFF1, mammaglobin A, FN, Epcam, tm4sf and rbpms is determined.

4. Method according to claim 3, for the preparation of an expression pattern used for tumor stage determination.

5. Method according to any of claims 1 to 4, wherein said patient is suffering from early stage cancer.

6. Method according to any one of claims 1 to 5 with a detection limit of less than 30 tumor cells/ml whole blood.

7. A multi-marker panel for detecting circulating tumor cells in a subject at risk of malignancy, consisting of the biomarkers CCNE2, DKFZp762E1312, EMP2, MAL2, PPIC, SLC6A8 and GTF2IRD1, and optionally one or more markers of the group consisting of AGR2, FXYD3, S100A16, TFF1, mammaglobin A, FN, Epcam, tm4sf and rbpms.

8. A set of reagents for detecting circulating tumor cells in a subject at risk of malignancy, consisting of reagents specifically binding to CCNE2, DKFZp762E1312, EMP2, MAL2, PPIC, SLC6A8 and GTF2IRD1, and optionally further one or more markers of the group consisting of AGR2, FXYD3, S100A16, TFF1, mammaglobin A, FN, Epcam, tm4sf and rbpms.

9. Set according to claim 8, wherein said reagents are ligands, such as antibodies or antibody fragments, which are optionally labelled.

## Patentansprüche

1. Verfahren zum Nachweisen von CCNE2 in einer Körperflüssigkeitsprobe eines Patienten zum Feststellen der Anfälligkeit für Brust-, Eierstock-, Endometrium- oder Gebärmutterhals-Krebs, umfassend
a. Testen der CCNE2-Nukleinsäureexpression in einer an Tumorzellen angereicherten Blutprobe von dem Patienten, und
b. Vergleichen des Ergebnisses mit einem Referenzspiegel, wobei ein erhöhter Spiegel ein Hinweis ist, dass der Patient ein Risiko für eine Tumorerkrankung aufweist, die Brustkrebs, Eierstockkrebs, Endometriumkrebs oder Gebärmutterhalskrebs ist.

2. Verfahren gemäß Anspruch 1, wobei eine vergleichende Genexpressionsanalyse durchgeführt wird.

3. Verfahren gemäß einem der Ansprüche 1 oder 2, wobei mindestens ein weiterer Marker bestimmt wird, der ausgewählt ist aus der Gruppe von DKFZp762E1312, EMP2, MAL2, PPIC, SLC6A8, GTF2IRD1, AGR2, FXYD3, S100A16, TFF1, Mammaglobin A, FN, EpCAM, TM4SF und RBPMS.

4. Verfahren gemäß Anspruch 3 für die Herstellung eines Expressionsmusters, das zur Tumorstadium-Bestimmung verwendet wird.

5. Verfahren gemäß einem der Ansprüche 1 bis 4, wobei der Patient an einem Karzinom im Frühstadium leidet.

6. Verfahren gemäß einem der Ansprüche 1 bis 5 mit einer Nachweisgrenze von weniger als 30 Tumorzellen/ml Vollblut.

7. Multi-Marker-Panel zum Nachweisen zirkulierender Tumorzellen in einem Individuum mit einem Risiko für eine maligne Erkrankung, bestehend aus den Biomarkern CCNE2, DKFZp762E1312, EMP2, MAL2, PPIC, SLC6A8 und GTF2IRD1 und gegebenenfalls einen oder mehrere Marker aus der Gruppe, bestehend aus AGR2, FXYD3, S100A16, TFF1, Mammaglobin A, FN, EpCAM, TM4SF und RBPMS.

8. Satz von Reagenzien zum Nachweisen zirkulierender Tumorzellen in einem Individuum mit einem Risiko für eine maligne Erkrankung, bestehend aus Reagenzien, die spezifisch an CCNE2, DKFZp762E1312, EMP2, MAL2, PPIC, SLC6A8 und GTF2IRD1 binden und gegebenenfalls ferner einen oder mehrere Marker aus der Gruppe, bestehend aus AGR2, FXYD3, S100A16, TFF1, Mammaglobin A, FN, EpCAM, TM4SF und RBPMS.

9. Satz gemäß Anspruch 8, wobei die Reagenzien Liganden sind, wie etwa Antikörper oder Antikörperfragmente, die gegebenenfalls markiert sind.

## Revendications

1. Procédé de détection de CCNE2 dans un échantillon de fluide corporel d'un patient pour détecter la susceptibilité envers le cancer du sein, le cancer des ovaires, le cancer endométrial, ou le cancer du col de l'utérus, comprenant les étapes consistant à
a. tester l'expression de l'acide nucléique CCNE2 dans un échantillon de sang enrichi en cellules tumorales dudit patient, et
b. comparer le résultat avec un niveau de référence, dans lequel un niveau élevé représente une indication sur le risque de contracter une maladie tumorale, qui est le cancer du sein, le cancer des ovaires, le cancer endométrial, ou le cancer du col de l'utérus.

2. Procédé selon la revendication 1, dans lequel on effectue une analyse comparative de l'expression génétique.

3. Procédé selon l'une quelconque des revendications 1 ou 2, dans lequel au moins un marqueur supplémentaire choisi parmi le groupe constitué de DKFZp762E1312, EMP2, MAL2, PPIC, SLC6A8, GTF2IRD1, AGR2, FXYD3, S100A16, TFF1, mammaglobine A, FN, Epcam, tm4sf et rbpms est déterminé.

4. Procédé selon la revendication 3, pour la préparation d'un motif d'expression utilisé pour la détermination du stade de la tumeur.

5. Procédé selon l'une quelconque des revendications 1 à 4, dans lequel ledit patient soufre de cancer à l'état précoce.

6. Procédé selon l'une quelconque des revendications 1 à 5 avec une limite de détection de moins de 30 cellules tumorales par ml de sang pur.

7. Panneau à marqueurs multiples pour la détection de cellules tumorales circulantes chez un sujet présentant un risque de malignité, constitué des biomarqueurs CCNE2, DKFZp762E1312, EMP2, MAL2, PPIC, SLC6A8 et GTF2IRD1, et éventuellement d'un ou de plusieurs marqueurs du groupe constitué de AGR2, FXYD3, S100A16, TFF1, mammaglobine A, FN, Epcam, tm4sf et rbpms.

8. Ensemble de réactifs pour la détection de cellules tumorales circulantes chez un sujet présentant un risque de malignité, constitué des réactifs se liant spécifiquement à CCNE2, DKFZp762E1312, EMP2, MAL2, PPIC, SLC6A8 et GTF2IRD1, et en outre éventuellement d'un ou de plusieurs marqueurs du groupe constitué de AGR2, FXYD3, S100A16, TFF1, mammaglobine A, FN, Epcam, tm4sf et rbpms.

9. Ensemble selon la revendication 8, dans lequel lesdits réactifs sont des ligands, tels que des anticorps ou des fragments d'anticorps, qui sont éventuellement marqués.
